# EUROPEAN PATENT SPECIFICATION

(11) **EP 1 501 571 B1**
(45) Date of publication and mention of the grant of the patent: **05.05.2010**
(21) Application number: 03708787.1
(22) Date of filing: 12.03.2003
(51) Int. Cl.: A61M 5/142

(54) **AMBULATORY INFUSION MEMBRANE PUMP**
AMBULANTE INFUSIONSMEMBRANPUMPE
POMPE A MEMBRANE POUR PERFUSION AMBULATOIRE

(30) Priority: 14.03.2002 SE 0200760
(43) Date of publication of application: 02.02.2005
(73) Proprietor: Nilson, Billy, S-595 52 Mjölby (SE); Medux AB, 595 30 Mjölby (SE)
(72) Inventor: Nilson, Billy, S-595 30 Mjölby (SE)
(74) Representative: Hammond, Andrew David
(86) International application number: PCT/SE2003/000411
(87) International publication number: WO 2003/075984

(56) References cited:
- WO-A1-99/21596
- WO-A2-02/04047
- US-A- 4 846 636
- US-A1- 2002 004 015

## Description

### TECHNICAL FIELD

The present invention relates to fluid injection pumps with disposable membrane pump and tubing intended for injection of medicines, pain relief and fluids related to nursing.

### BACKGROUND OF THE INVENTION

Membrane injection pumps are used in many applications, as ultra small plastic pumps intended for medical applications. However, when used for portioning fluids, as medicines, with a high degree of precision, usually other pumping methods dominate the market. Such pumps are syringe or plunger pumps, as well as peristaltic pumps.

Transporting fluids from a container to an injection needle with a high degree of precision is claimed as to the dosage, especially concerning new, potent medicines. Not intended leakage through tubing arrangement must be safely restricted, as when a container filled with medicine is placed above the needle, the static pressure can generate a flow if the system is not restricted against this.

Examples of prior art are:
US 6,261,066, US 6,234,992, US 5,533,886, US 6,203,291, WO 0028213, US 5,478,211, US 4,838,860, US 5,637,093, US 4,898,579

Previously infusion pumps for supplying fluids to patients were usually syringe type, peristaltic type, membrane type or plunge pumps. Many of these pumps are very sophisticated and sometimes very complicated, as US 5,482,438 and very expensive for the hospitals. Others are simplified as US 6,203,291, using an oscillating movement from semiconductors affecting a diaphragm at resonance frequency and with diffusers as controlling means in the inlet and outlet areas. Another invention uses semiconductors in a micro membrane pump, as in US 6,261,06. US 5,368,570 is a plurality of membrane pumps.

US 4,898,579 is a dual chamber piston and cylinder pump which uses a disposable cassette and with two cylinders can achieve an almost uninterrupted fluid feeding. However the cassette is with its arrangements of check valves a little bit complicated, and the amount of material in the cassette is significantly high. When initially filling up the pump, de-airing obviously will become problematic.

Document US 4 846 636 describes a piston pump with a complicated construction, where it is very difficult to de-air a pumped fluid in a controlled way. The design of this pump requires sensors for detecting air in the fluid. The check valves of this pump are further built-in into the pump house of the piston pump.

In document WO 99/21596 there is disclosed an infusion pump according to the pre-amble of claim 1 with check valves built-in into the pump house, whereby there are also in this disclosure small possibilities to watch air in the pump system. This pump has a large dead space, which means that a substantial amount of fluid is interlocked into the pump when a pumping operation is ended. The principle of this infusion pump is based on a pumping mechanism using a semi-membrane and a semi-piston.

A careful control of the pumped amount of fluid by use of different piston stroke lengths are not possible in any of the designs of US 4 846 636 and WO 99/21596.

None of the preceding pumps unites disposability together with high precision dosages, safety against leakage, material saving and an uncomplicated concept as in our invention.

### SUMMARY OF THE INVENTION

The present invention is a membrane pump as defined in claim 1. Preferably it is consisting of two main bodies: the computer with its housing and the administration set. The membrane pump works distinctly with equal membrane movements repeated as a decided position of the stroke. A rotational electric motor with a gear is positioned in the housing, and the outgoing shaft moves a screw in connection with a threaded plunger rod and at the other end in connection with the membrane tap. At the end cover on the housing the membrane pump is snapped into an attachment consisting of a guide rail and a shoulder on the pump house, which easily is snapped on when the pump house is in exact position. The electrical motor has a permanent magnet and a sensor which registrates each revolution of the motor and communicates signals to the computer, which registers each signal in forward respectively backward mode of the driving motor, thus counting each revolution and registers the exact position of the screw on the plunger rod.

With a high revolution electrical motordrive connected to a gear the advance of the screw works in a very high degree of precision, independent of accidental wear. Accordingly the membrane moves with exact precision accordingly to ordered revolutions rolled by the electrical motor, and the dosage can be controlled with each stroke of the plunger rod.

In a system with tubes and pump, elastic effects can arise, for instance when the pump suddenly is cut off (which happens when the polarity reversal activates for intended reversal of the plunger rod). When the pressure leaves the system, elastic effects arise; the volume of the system decreases. In order to prevent unintended through flow in the system according to such elastic effects, check valves are positioned in each end of the tubing; the inflow check valve close to the fluid container and the outflow check valve close to the needle arrangement.

Visual control of the pump function occurs with transparent valve housing at the inflow check valve with a colored cone indicating the working of the pump and visible at a distance of at least some meters.

The-to-and fro movement of the pump is driven by an electric motor in connection with a screw-driven feed. Making the pitch optimal, the feed becomes very accurate; e.g. when back-feeding through reversing of the electric motor by reverse polarity, it is very important that the movement is under strict control and not too fast, when gas otherwise could become extricated out of the fluid (cavitation). A processor is used to control the motor drive. The processor has a control puls circuit which reverses the motor drive by reverse polarity. The processor registers the working speed and controls the ordered revolutions and when exact feeding distance of the piston is achieved. The movement of the piston and as well pumped quantity is thus controlled in both directions with high degree of precision.

The processor has a box with a key set and a display intended for controlling the dosage and watching different functions as for the safety. The box also has rechargeable batteries. A patient needing fluid, drugs or pain killers by infusion, can carry the complete pump set conveniently and freely move him without handling a stand, used for the drip chamber. Our pump does not need the static pressure from the height with which the fluid container is held by the stand. The pump generates this pressure by pumping. Thus it is suitable to place the fluid container where it is convenient.

Instead of counting the drips in the drip chamber and throttling the tubing, you just order right dosage on display by using the keys. If you want to watch the pump working , you look at the transparent check valve house close to the fluid container and at the pulsating, abovementioned cone. A more advanced model you order the dosage with a bar code and a bar code reader.
When ambulance transports are acquired, as by accidents or transporting patients, our device is even handier because of independence of a stand. The device can also be used positioned on the side of a patient in bed.

An infusion pump must be reliable and stop working, if divergence from normal working happens, e.g. if the fluid encounters higher or lower resistance and the pump thus meets occlusion or very low pressure e.g. when air is in the system. Those circumstances must be readable and the pump must stop if not allowed deviations happen. If such deviations happen, variations of the motor current is watched by the control system and as the electric motor drive is pulse driven, the relations between pulses, current and revolution is programmed in the control system, which signals and stops the motor drive within certain limits. The display writes which kind of deviation happened. Pressure sensors are not necessary to use to watch the correct working of the pump.

If gases or air are present in the tube or pump regions, the inlet check valve has measures for de-airing the system and press the air back to the liquid container. In a preferred achievement the cone of the check valve is hollow and floats on the liquid where-by it presses the air back through the seat face. The last amount of air leaks back to the liquid container by a slit on the cone, which slit is wide enough for gaseous fluids, but to narrow for liquids to pass. The system is de-aired. When larger amounts of air is present, as with glass containers, an outlet with an air filter is arranged in the housing of the inlet check valve.

The precision of the dosage of the membrane pump is not restricted because of small dimensions, which precision becomes extremely high compared to e.g. a peristaltic pump, which type is most frequently used with ambulatory pumps. The pump house and the details of the administration set is in a preferred accomplishment made of as drug certified thermo plastic, produced with precision in many units.

The administration set together with the liquid (medicine) container is disposable, as with the present drip chamber. When the liquid is emptied, the administration set is disposed of, and a new set is in use, when next batch is inserted. The liquid container usually is collapsible, but a stiff one as made of glass is also used and are united with the administration set in the same way as with drip chambers.

All types of fluids are possible to introduce into the patient, frequently intravenously or subcutaneously, but also in body openings or against the body, the skin or mucous membranes. With a bar code system you can programme the control unit so as to introduce the right kind and amount of drug. The membrane pump has a wide use: providing drugs, pain killers, nutrition solution or liquid.

The invention has many other applications, other than drugs. The pump is useful as a distributor of chemical-technical fluids and pastes if in right dimensions and the same design. The same material, injection molded plastic, which is recyclable, is used.

### BRIEF DESCRIPTION OF THE DRAWINGS

FIG. 1 is a side-sectional view of the embodiment with insert mouthpiece for the liquid container, inlet check valve, inlet tube, membrane pump with box and feed screw with electric motor, outlet tube with attachment to the needle and needle.
FIG. 2 is the outlet check valve, needle attachment and needle.
FIG. 3 is the pump house with membrane, inlet and outlet.
FIG. 4 is an assembly of the device box and the administration set.

### DESCRIPTION OF THE PREFERRED EMBODIMENT

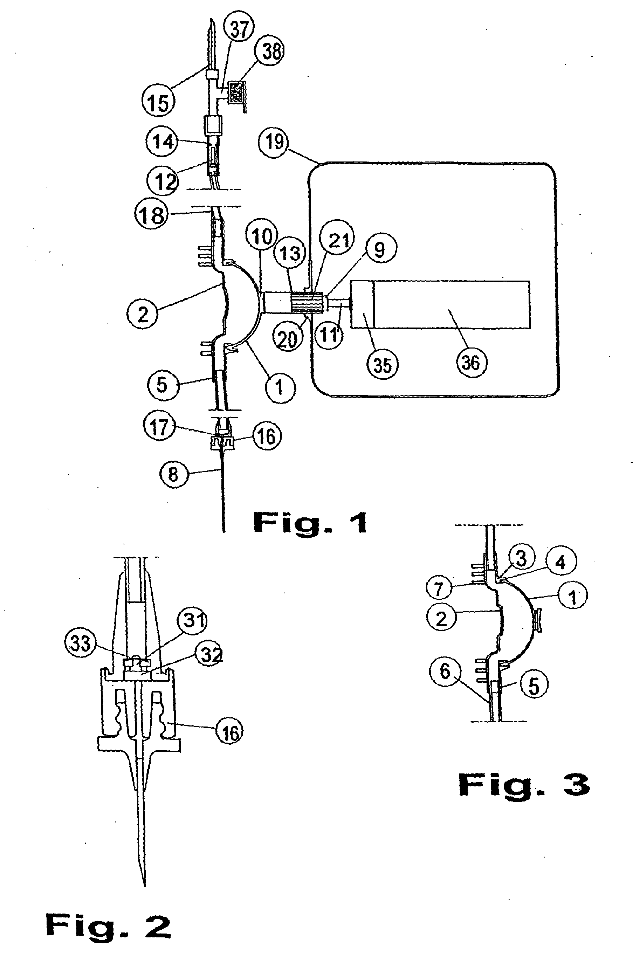

The exemplary embodiment of the membrane pump of the present invention, indicated generally as the pump house 2 in FIG. 1 with the membrane 1 and connected to an inlet tube 18 and outlet tube 5, with inlet check valve14 and outlet check valve 16, and an attachment to the injection needle 8. The liquid container is penetrated by the insert mouthpiece 15 which is connected to the inlet check valve house 14 and with a cone 12, which floats, movable longitudinally in the house 14. The air filter 38 is connected to the inlet tube 15. The device box 19 is schematic drawn and contains an electric motor 36 with gear box 35 and a screw 11 which when rotated moves the threaded piston 9 longitudinally and the elongation of the piston 13 which presses the membrane inwards and lets the membrane 1 free for recoiling , whereas the membrane pump is filled with liquid. The elongation 13 of the piston 9 has splines 21 which fit in corresponding tapping in the gablesocket 20 in the device box 19 and locks the piston from rotation when the screw rotates.

The check valve with housing 16 in FIG. 2, with a perforated disk 32 with a nib 31 in the center, on which the check valve membrane 33 is clamped up. By making different thicknesses of the membrane 33, and different preloads to the seat, you can control at which pressure the check valve membrane 33 will open, which is important so as not to leak when a high static pressure arises from a high positioned liquid container.

In FIG. 3 is shown details in the pump house 2 with a weldable attachment 4 of the membrane 1 to a part 3 of the pump house 2. The outlet tube 6 is welded to the outlet 5 of the pump. The circular flanges 7 are intended to increase the strength of the removable attachment to the device box 19.

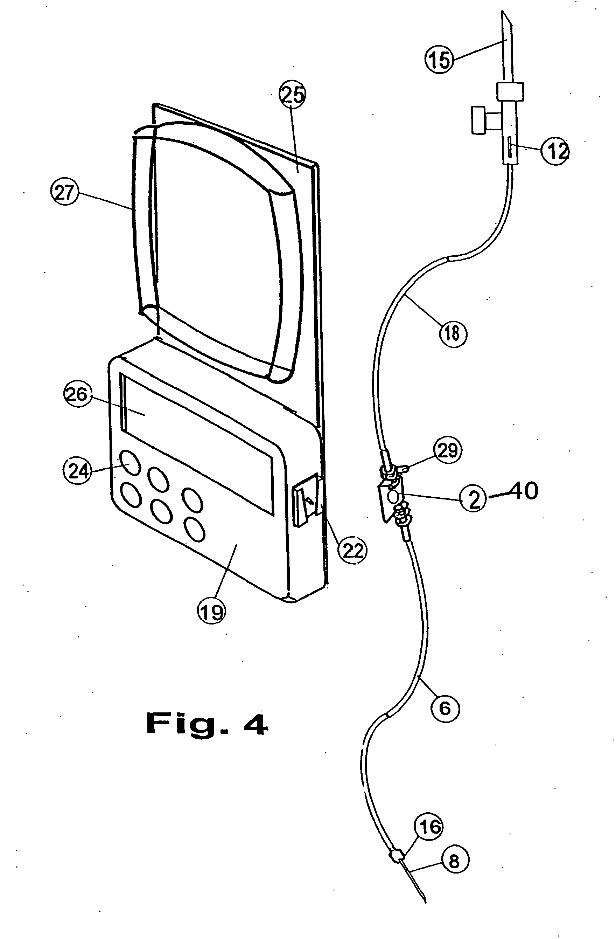

The membrane pump system is shown in FIG.4 with the device box 19 and the administration set 40. The device box 19 consists of a display 26, keys 24, a rapid attachment 22 of the pump house 2, a body 25 to the liquid container 27, here shown as a collapsible container. The administration set 40 has a hold 29 intended for attaching and stop against the grip 22, and in the upper part a penetrating inlet 15, a check valve cone 12, a tube 18, a pump 2 and an outlet tube 6 down to the outlet check valve 16 and injection needle 8.

## Claims

1. A membrane pump intended for transporting a controlled average of volumetric flow rate of a fluid from a fluid source through an outlet to a patient, comprising:
- a pump house (2) with a membrane (1),
- a needle arrangement (8),
- an inlet flexible tube (18) connected to an inlet of said pump house (2),
- an outlet flexible tube (6) connected to an outlet (5) of said pump house (2),
**characterized in that**
- an inlet check valve (14) is connected to the end of said inlet tube (18) positioned closest to the fluid source, and
- an outlet check valve (16) is connected to the end of said outlet tube (6) positioned close to said needle arrangement (8).

2. The membrane pump according to claim 1, further comprising:
(a) said inlet (18) and outlet tubes (6) having inner diameters between 0,4 and 2 mm,
(b) a box (19) with an electric motor (36), wherein the pump house (2) of the membrane pump is adapted to be docked to the box (19), and
(c) electric control means including the electric motor (36), a gear (35), a feeding screw (11), a nut and a piston (9) for controlling the average volumetric flow rate by means of a control of the forward and backward movement of the piston (9).

3. The membrane pump according to claim 2, wherein:
- said membrane (1) is yieldable and dimensioned for safely back springing and thus suction of the fluid into the pump house (2),
- a boss (10) provided with grooves is arranged on the centre of the membrane (1), and said piston (9) provided with a yoke gripping into said grooves and thus mechanically drives the membrane (1) forwards and backwards.

4. The membrane pump according to claim 3, wherein the electric motor (36) and the gear (35) are connected with the feeding screw (11) and a threaded nut in combination with the piston (9) for accomplishing the forward and backward movement, and wherein the piston (9) is provided with splines (21) fitting into an end plate (20) of the box (19) and wherein the piston (9) is in contact with said boss of the membrane (1) when the pump house (2) is mounted on the box (19).

5. The membrane pump according to claim 4, wherein the electric motor (36) has a revolution registering device connected to a control circuit, wherein said control circuit is provided with a display (26) and keys, for operating said feeding screw (11) to order a decided stroke of the piston (9) and said stroke being repeated until a new order is received from the control circuit, whereby the volumetric flow pumped with each stroke is controlled.

6. The membrane pump according to claim 5, wherein said control circuit registers the current during a decided short time interval of normal pumping and when the current significantly deviates, the control circuit stops the pumping and gives a signal and an information on the display (26).

7. The membrane pump according to claim 6, wherein the control circuit is programmable with a dosage of the transporting and with pump work time intervals,

8. The membrane pump according to claim 7, wherein the box is provided with a bar code reader and the control circuit being programmed for medicines and dosages, safeguarding that right data are programmed into the pump.

9. The membrane pump according to claim 8, wherein the box (19) is provided with rechargeable batteries driving the controls and the electric motor (36).

10. The membrane pump according to claim 1, wherein the inlet check valve (14) has a cone (12), which is indented with a cut, 0,1 to 0,5 mm deep, in the upper part so that the valve (14) is pervious when gaseous fluid occurs, but tight when liquid fluid occurs.

11. The membrane pump according to claim 1 or 10, wherein the inlet check valve (14) has a coloured valve cone (12) and that said inlet check valve (14) is provided with a transparent housing, so that said coloured valve cone (12) is easy to observe.

12. The membrane pump according to claim 10 or 11, wherein said cone (12) of the inlet check valve (14) has minor lugs, which affect the cone (12) to move precisely in said transparent housing and that said cone (12) has a lower density than the fluid pumped for effectively de-air liquid containing air or other gases.

13. The membrane pump according to any one of claims 10 to 12, wherein said cone (12) is made to float on said liquid, whereby the cone and the fluid presses air back through a seat face of the inlet valve (14), if air is present in the liquid.

## Patentansprüche

1. Membranpumpe zum Transportieren eines kontrollierten durchschnittlichen Volumenstroms eines Fluids von einer Fluidquelle durch einen Auslass zu einem Patienten, umfassend
- ein Pumpengehäuse (2) mit einer Membran (1)
- eine Nadelanordnung (8)
- eine flexible Einlassröhre (18), die mit einem Einlass des Pumpengehäuses (2) verbunden ist
- eine flexible Auslassröhre (6), die mit einem Auslass (5) des Pumpengehäuses (2) verbunden ist,
**dadurch gekennzeichnet, dass**
- ein einlassseitiges Rückschlagventil (14) mit dem Ende der Einlassröhre (18) verbunden und nahe der Fluidquelle angeordnet ist und dass
- ein auslassseitiges Rückschlagventil (16) mit dem Ende der Auslassröhre (6) verbunden und in der Nähe der Nadelanordnung (8) angeordnet ist.

2. Membranpumpe nach Anspruch 1, **dadurch gekennzeichnet, dass** sie weiter umfasst:
(a) die Einlassröhre (18) und Auslassröhren (6) mit Innendurchmessern zwischen 0,4 und 2 mm,
(b) einen Kasten (19) mit einem elektrischen Motor (36), wobei das Pumpengehäuse (2) daran angepasst ist, an den Kasten (19) angedockt zu werden, und
(c) elektrische Steuerungsmittel, umfassend den elektrischen Motor (36), ein Getriebe (35), eine Förderspindel (11), eine Mutter und einen Kolben (9) zum Steuern des durchschnittlichen Volumenstroms mittels einer Steuerung der Vor- und Zurückbewegung des Kolbens (9).

3. Membranpumpe nach Anspruch 2, **dadurch gekennzeichnet, dass**
- die Membran (1) dehnbar und so dimensioniert ist, dass sie sicher zurückfedert und somit das Fluid in das Pumpengehäuse (2) saugt,
- auf der Mitte der Membran (1) ein mit Rillen versehener Vorsprung (10) vorgesehen ist und der mit einem Kopf versehene Kolben (9) in die Rillen greift und somit die Membran (1) mechanisch vor und zurück treibt.

4. Membranpumpe nach Anspruch 3, **dadurch gekennzeichnet, dass** der elektrische Motor (36) und das Getriebe (35) mit der Förderspindel (11) und einer Gewindemutter in Kombination mit dem Kolben (9) verbunden sind, um die Vorwärts- und Rückwärtsbewegung zu erzielen, und dass der Kolben (9) mit geometrischen Ausprägungen (21) versehen ist, die in eine Endplatte (20) des Kastens (19) greifen, und dass der Kolben (9) in Kontakt mit dem Vorsprung der Membran (1) steht, wenn das Pumpengehäuse (2) auf dem Kasten (19) montiert ist.

5. Membranpumpe nach Anspruch 4, **dadurch gekennzeichnet, dass** der elektrische Motor (36) mit einer Vorrichtung zum Feststellen der Umdrehungen versehen ist, die mit einem Steuerschaltkreis verbunden ist, wobei der Steuerschaltkreis mit einer Anzeige (26) und Tasten versehen ist, um die Förderspindel (11) zu bedienen, um einen bestimmten Hub des Kolbens (9) auszulösen, der wiederholt wird, bis von dem Steuerschaltkreis ein neuer Befehl erhalten wird, wodurch der mit jedem Hub gepumpte Volumenstrom gesteuert wird.

6. Membranpumpe nach Anspruch 5, **dadurch gekennzeichnet, dass** der Steuerschaltkreis während einer bestimmten kurzen Zeitspanne beim normalen Pumpen den Strom feststellt, und dass, wenn der Strom deutlich abweicht, der Steuerschaltkreis das Pumpen anhält und ein Signal und eine Information auf der Anzeige (26) ausgibt.

7. Membranpumpe nach Anspruch 6, **dadurch gekennzeichnet, dass** der Steuerschaltkreis mit einer Dosis für den Transport und mit Zeitintervallen für den Betrieb der Pumpe programmierbar ist.

8. Membranpumpe nach Anspruch 7, **dadurch gekennzeichnet, dass** der Kasten mit einem Strichcode-Leser versehen ist und der Steuerschaltkreis hinsichtlich Medikamenten und Dosierungen programmiert ist, wodurch sichergestellt wird, dass korrekte Daten in die Pumpe programmiert werden.

9. Membranpumpe nach Anspruch 8, **dadurch gekennzeichnet, dass** der Kasten (19) mit wieder aufladbaren Batterien versehen ist, welche die Steuerung und den elektrischen Motor (36) versorgen.

10. Membranpumpe nach Anspruch 1, **dadurch gekennzeichnet, dass** das einlassseitige Rückschlagventil (14) einen Kegel (12) aufweist, in dem im oberen Teil ein Einschnitt mit einer Tiefe von 0,1 bis 0,5 mm vertieft ist, so dass das Ventil (14) durchlässig ist, wenn gasförmiges Fluid auftritt, aber dicht, wenn flüssiges Fluid auftritt.

11. Membranpumpe nach einem der Ansprüche 1 oder 10, **dadurch gekennzeichnet, dass** das einlassseitige Rückschlagventil (14) einen eingefärbten Ventilkegel (12) aufweist und dass das einlassseitige Rückschlagventil (14) mit einem transparenten Gehäuse versehen ist, so dass der eingefärbte Ventilkegel (12) einfach beobachtet werden kann.

12. Membranpumpe nach einem der Ansprüche 10 oder 11, **dadurch gekennzeichnet, dass** der Kegel (12) des einlassseitigen Rückschlagventils (14) kleinere Ansätze aufweist, die den Kegel (12) dahingehend beeinflussen, dass er sich in dem transparenten Gehäuse präzise bewegt, und dass der Kegel (12) eine geringere Dichte aufweist als das gepumpte Fluid, um wirksam eine Flüssigkeit, die Luft oder andere Gase enthält, zu entgasen.

13. Membranpumpe nach einem der Ansprüche 1 bis 12, **dadurch gekennzeichnet, dass** der Kegel (12) dazu gebracht wird, auf der Flüssigkeit zu schwimmen, wodurch der Kegel und das Fluid Luft zurück durch eine Sitzfläche des einlassseitigen Ventils (14) drücken, falls in der Flüssigkeit Luft vorhanden ist.

## Revendications

1. Pompe à membrane destinée au transport d'une moyenne commandée d'un débit volumétrique d'un fluide depuis une source de fluide à travers une sortie jusqu'à un patient, comprenant :
- un carter de pompe (2) avec une membrane (1),
- un ensemble d'aiguille (8),
- un tube flexible d'entrée (18) relié à une entrée dudit carter de pompe (2),
- un tube flexible de sortie (6) relié à une sortie (5) dudit carter de pompe (2),
**caractérisée en ce que**
- un clapet antiretour d'entrée (14) est relié à l'extrémité dudit tube d'entrée (18) positionné le plus près de la source de fluide et
- un clapet antiretour de sortie (16) est relié à l'extrémité dudit tube de sortie (6) positionné près dudit ensemble d'aiguille (8).

2. Pompe à membrane selon la revendication 1, comprenant en outre :
(a) ladite entrée (18) et les tubes de sortie (6) avec des diamètres intérieurs compris entre 0,4 et 2 mm,
(b) une boîte (19) avec un moteur électrique (36), le carter de pompe (2) de la pompe à membrane étant adapté de sorte à être arrimé à la boîte (19), et
(c) des moyens de commande électrique comportant le moteur électrique (36), un engrenage (35), une vis d'alimentation (11), un écrou et un piston (9) pour la commande du débit volumétrique moyen par l'intermédiaire d'une commande du mouvement vers l'avant et vers l'arrière du piston (9).

3. Pompe à membrane selon la revendication 2, dans laquelle :
- ladite membrane (1) est déformable et dimensionnée pour la détente en toute sécurité et ainsi l'aspiration du fluide dans le carter de pompe (2),
- un bossage (10) doté de gorges est disposé au centre de la membrane (1) et le dit piston (9) doté d'un étrier vient en prise dans lesdites gorges et conduit ainsi mécaniquement la membrane (1) vers l'avant et vers l'arrière.

4. Pompe à membrane selon la revendication 3, dans laquelle le moteur électrique (36) et l'engrenage (35) sont reliés à la vis d'alimentation (11) et un écrou fileté en combinaison avec le piston (9) pour accomplir le mouvement vers l'avant et vers l'arrière, et dans laquelle le piston (9) est doté de cannelures (21) s'adaptant dans une plaque d'extrémité (20) de la boîte (19) et
dans laquelle le piston (9) est en contact avec ledit bossage de la membrane (1) lorsque le carter de pompe (2) est monté sur la boîte (19).

5. Pompe à membrane selon la revendication 4, dans laquelle le moteur électrique (36) a un dispositif compte-tours relié à un circuit de commande, dans laquelle ledit circuit de commande est doté d'un dispositif d'affichage (26) et de clés pour le fonctionnement de ladite vis d'alimentation (11) pour ordonner un course décidée du piston (9) et ladite course étant répétée jusqu'à ce qu'un nouvel ordre soit reçu du circuit de commande, moyennant quoi le débit volumétrique pompé à chaque course est commandé.

6. Pompe à membrane selon la revendication 5, dans laquelle ledit circuit de commande enregistre le courant pendant un bref intervalle de temps décidé de pompage normal et lorsque le courant dévie de manière significative, le circuit de contrôle arrête le pompage et donne un signal et une information sur le dispositif d'affichage (26).

7. Pompe à membrane selon la revendication 6, dans laquelle le circuit de commande est programmable avec un dosage de transport et avec des intervalles de temps de travail de la pompe.

8. Pompe à membrane selon la revendication 7, dans laquelle la boîte est équipée d'un lecteur de code-barres et le circuit de commande étant programmé pour des médicaments et des dosages, garantissant que des données correctes sont programmées dans la pompe.

9. Pompe à membrane selon la revendication 8, dans laquelle la boîte (19) est équipée de batteries rechargeables actionnant les commandes et le moteur électrique (36).

10. Pompe à membrane selon la revendication 1, dans laquelle le clapet antiretour d'entrée (14) présente un cône (12) qui est dentelé avec une coupe de 0,1 à 0,5 mm de profondeur dans la partie supérieure de sorte que le clapet (14) soit perméable lorsque le fluide gazeux apparaît mais imperméable lorsque le fluide liquide apparaît.

11. Pompe à membrane selon la revendication 1 ou 10, dans laquelle le clapet antiretour d'entrée (14) présente un cône de clapet coloré (12) et ledit clapet antiretour d'entrée (14) est équipé d'un logement transparent de sorte que ledit cône de clapet (12) coloré soit facile à observer.

12. Pompe à membrane selon la revendication 10 ou 11, dans laquelle ledit cône (12) du clapet antiretour d'entrée (14) présente de petites oreilles qui affectent le cône (12) pour se déplacer précisément dans ledit logement transparent et ledit cône (12) présente une densité inférieure à celle du fluide pompé pour désaérer effectivement du liquide contenant de l'air ou d'autres gaz.

13. Pompe à membrane selon l'une quelconque des revendications 10 à 12, dans laquelle ledit cône (12) est fabriqué pour flotter sur ledit liquide, moyennant quoi le cône et le fluide repressent l'air par une face de siège du clapet antiretour (14) si l'air est présent dans le liquide.
